# EUROPÄISCHE PATENTSCHRIFT

(11) **EP 3 791 174 B1**
(45) Veröffentlichungstag und Bekanntmachung des Hinweises auf die Patenterteilung: **02.07.2025**
(21) Anmeldenummer: 19722840.6
(22) Anmeldetag: 03.05.2019
(51) Int. Cl.: G01N 31/12, G01N 33/00

(54) **VERWENDUNG EINER ZUFUHREINRICHTUNG ZUM EINFÜHREN VON PROBEN IN EINE VORRICHTUNG**
USE OF A SUPPLY DEVICE FOR INTRODUCING SAMPLES INTO A DEVICE
UTILISATION D'UN DISPOSITIF D'ALIMENTATION POUR INTRODUIRE DES ÉCHANTILLONS DANS UN DISPOSITIF

(30) Priorität: 08.05.2018 DE 102018207165
(43) Veröffentlichungstag der Anmeldung: 17.03.2021
(73) Patentinhaber: GEOMAR Helmholtz Centre for Ocean Research Kiel, 24148 Kiel (DE)
(72) Erfinder: HANSEN, Thomas, 24220 Flintbek (DE)
(74) Vertreter: Wallinger Ricker Schlotter Tostmann
(86) Internationale Anmeldenummer: PCT/EP2019/061336
(87) Internationale Veröffentlichungsnummer: WO 2019/215021

(56) Entgegenhaltungen:
- EP-A1- 3 193 165
- DE-A1- 102006 015 535
- DE-A1- 102013 019 697
- DE-U1- 202004 015 247
- FR-A1- 2 734 363
- US-A1- 2016 231 298
- NATACHA GENTILE ET AL: "delta15N measurement of organic and inorganic substances by EA-IRMS: a speciation-dependent procedure", ANALYTICAL AND BIOANALYTICAL CHEMISTRY, vol. 405, no. 1, 26 October 2012 (2012-10-26), pages 159 - 176, XP035159160, ISSN: 1618-2650, DOI: 10.1007/S00216-012-6471-Z
- HANS-PETER SIEPER ET AL: "A measuring system for the fast simultaneous isotope ratio and elemental analysis of carbon, hydrogen, nitrogen and sulfur in food commodities and other biological material", RAPID COMMUNICATIONS IN MASS SPECTROMETRY, vol. 20, no. 17, 31 July 2006 (2006-07-31), pages 2521 - 2527, XP055020966, ISSN: 0951-4198, DOI: 10.1002/rcm.2619
- MATTHIAS GEHRE ET AL: "High-temperature elemental analysis and pyrolysis techniques for stable isotope analysis", RAPID COMMUNICATIONS IN MASS SPECTROMETRY, vol. 17, no. 13, 15 July 2003 (2003-07-15), pages 1497 - 1503, XP055117270, ISSN: 0951-4198, DOI: 10.1002/rcm.1076

## Beschreibung

Die vorliegende Erfindung betrifft eine Verwendung einer Zufuhreinrichtung zum Einführen von Proben in eine Vorrichtung zum Behandeln von Proben, insbesondere Kleinstproben, für eine quantitative Elementaranalyse bezüglich Kohlenstoff, Stickstoff und/oder Schwefel.

Aus dem Stand der Technik der DE 101 51 646 A1 ist eine CNS Isotopenanalytik mit zwei Trennsäulen mit Ventilschaltung bekannt. Weiter zeigt die DE 102 35 662 A1 ein Probenaufgabesystem. Allen derzeit bekannten Analysesystemen zur CNS Isotopenanalytik ist gemein, dass die simultane CN oder CNS Messung von CNS Kleinstproben weder sequentiell noch simultan möglich ist.

Der Artikel "δ¹⁵N measurement of organic and inorganic substances by EA-IRMS: a speciation-dependent procedure" von NATACHA GENTILE ET AL, veröffentlicht am 26. Oktober 2012 in ANALYTICAL AND BIOANALYTICAL CHE-MISTRY, Bd. 405, Nr. 1, Seiten 159-176, offenbart eine Vorrichtung und ein Verfahren zur Messung von δ¹⁵N von organischen und anorganischen Substanzen, bei dem eine Probe unverzüglich bei ca. 1700°C in gasförmige Verbrennungsprodukte umgewandelt wird, welche durch eine PoropaQ Gaschromatograph-Säule getrennt werden, und anschließend in einem Massenspektrometer aufgetrennt und analysiert werden.

Weiterer Stand der Technik ist aus den Druckschriften DE 20 2004 015247 U1, DE 10 2013 019697 A1, DE 10 2006 015535 A1, FR 2 734 363 A1,

EP 3 193 165 A1 und US 2016/231298 A1 sowie den Artikeln "A measuring system for the fast simultaneous isotope ratio and elemental analysis of carbon, hydrogen, nitrogen and sulfur in food commodities and other biological material" von HANS-PETER SIEPER ET AL, veröffentlicht am 31. Juli 2006 in RAPID COMMUNICATIONS IN MASS SPECTROMETRY, Bd. 20, Nr. 17, Seiten 2521-2527, und "High-temperature elemental analysis and pyrolysis techniques for stable isotope analysis" von Matthias Gehre ET AL, veröffentlicht am 15. Juli 2003 in Rapid Communications in Mass Spectrometry, Bd. 17, Nr. 13, Seiten 1497-1503 bekannt.

Es ist eine Aufgabe der Erfindung, eine effiziente Elementaranalyse von C, N und/oder S, mittels der Kleinstproben analysierbar sind, zu ermöglichen.

Diese Aufgabe wird durch die Merkmale des Patentanspruchs 1 gelöst. Weitere bevorzugte Ausführungsformen der Erfindung sind Gegenstand der abhängigen Ansprüche.

Aspekte der Erfindung werden nachfolgend dargestellt:
Eine nicht beanspruchte Vorrichtung zur Behandlung von Proben, insbesondere Kleinstproben, für eine Trennung in einem Gaschromatographen zur Elementaranalyse bezüglich Kohlenstoff, Stickstoff und/oder Schwefel, weist eine erste Behandlungseinrichtung mit einer Öffnung auf, wobei in der ersten Behandlungseinrichtung eine der ersten Behandlungseinrichtung über die Öffnung zugeführte Probe zumindest teilweise verdampft werden kann und die dazu eingerichtet ist, die zumindest teilweise verdampfte Probe in einem ersten Oxidationsbereich erstmalig zu oxidieren, in einem Reduktionsbereich erstmalig zu reduzieren und in einem zweiten Oxidationsbereich zweitmalig zu oxidieren, wobei der Reduktionsbereich zwischen dem ersten und zweiten Oxidationsbereich angeordnet ist.

Eine zweite Behandlungseinrichtung ist mit der ersten Behandlungseinrichtung verbunden und dazu eingerichtet, die in der ersten Behandlungseinrichtung zumindest teilweise verdampfte Probe zweitmalig zu reduzieren.

Eine nicht beanspruchte Zufuhreinrichtung zum Einführen von Proben, insbesondere Kleinstproben, in eine Vorrichtung zur Behandlung von Proben weist eine Eintrittsöffnung, über welche die Zufuhreinrichtung mit einer Zufuhrvorrichtung zur Bereitstellung von Proben, insbesondere Kleinstproben, koppelbar ist, und eine Austrittsöffnung, über welche die Zufuhreinrichtung mit der Vorrichtung verbindbar ist, auf. Die Zufuhreinrichtung ist dazu eingerichtet, eine mittels der Zufuhrvorrichtung über die Eintrittsöffnung eingeführte Probe entlang einer Längsachse durch einen Heizbereich zu führen, so dass die Probe beim Durchfallen der Zufuhreinrichtung vor Austritt durch die Austrittsöffnung zumindest teilweise verdampft wird.

Gemäß einem Aspekt der Erfindung wird bei der Verwendung einer Zufuhreinrichtung zum Einführen von Proben, insbesondere Kleinstproben, in eine Vorrichtung zur Behandlung von Proben, und vorzugsweise zum zumindest teilweisen Verdampfen der Proben beim Einführen in eine solche Vorrichtung, die Zufuhreinrichtung in Abhängigkeit von der Probenmenge und/oder mindestens einer Probeneigenschaft aus mehreren Zufuhreinrichtungen mit verschiedenen Durchmessern und/oder verschiedenen Längen gewählt.

Bei einem nicht beanspruchten Verfahren zur quantitativen Elementaranalyse von Proben, insbesondere Kleinstproben, bezüglich Kohlenstoff, Stickstoff und/oder Schwefel, wird eine Probe für eine Trennung in einem Gaschromatographen behandelt und die so zumindest teilweise verdampften Probe in dem Gaschromatographen in mindestens zwei Teilgase getrennt. Ein erstes Referenzgas wird in ein Massenspektrometer eingeleitet und ein entsprechend vom Massenspektrometer erzeugtes erstes Referenzsignal gemessen. Zudem wird ein erstes der mindestens zwei Teilgase aus dem Gaschromatographen in das Massenspektrometer eingeleitet und ein entsprechend erzeugtes erstes Probensignal gemessen. Weiterhin wird ein zweites Referenzgas in das Massenspektrometer eingeleitet und ein entsprechend vom Massenspektrometer erzeugtes zweites Referenzsignal gemessen. Des Weiteren wird ein zweites der mindestens zwei Teilgase aus dem Gaschromatographen in das Massenspektrometer eingeleitet und ein entsprechend erzeugtes zweites Probensignal gemessen. Vorzugsweise wird auch ein drittes Referenzgas in das Massenspektrometer eingeleitet und ein entsprechend vom Massenspektrometer erzeugtes drittes Referenzsignal gemessen. Zudem wird in bevorzugter Weise ein drittes der mindestens zwei Teilgase aus dem Gaschromatographen in das Massenspektrometer eingeleitet und ein entsprechend erzeugtes drittes Probensignal gemessen. Zudem wird der Kohlenstoff-, Stickstoff- und/oder Schwefelgehalt der Probe anhand des ersten und zweiten, vorzugsweise auch anhand des dritten, Referenzsignals und des ersten und zweiten, vorzugsweise auch anhand des dritten, Probensignals bestimmt.

Ein nicht beanspruchtes System zur quantitativen Elementaranalyse von Proben, insbesondere Kleinstproben, bezüglich Kohlenstoff, Stickstoff und/oder Schwefel, weist eine Zufuhrvorrichtung, insbesondere mit einer Zufuhreinrichtung, zum Bereitstellen einer Probe und eine Vorrichtung zum Behandeln der bereitgestellten, insbesondere mittels der Zufuhreinrichtung zugeführten, Probe auf. Zudem weist das System einen Gaschromatographen zum Trennen der zumindest teilweise verdampften Probe in mindestens zwei Teilgase und eine Gaszufuhrschnittstelle zum Steuern eines Gasflusses der mindestens zwei Teilgase aus dem Gaschromatographen und/oder von mindestens zwei Referenzgasen auf. Ein Massenspektrometer ist eingerichtet zum Ausgeben eines ersten und zweiten Referenzsignals, wenn dem Massenspektrometer ein erstes bzw. zweites Referenzgas mittels der Gaszufuhrschnittstelle zugeführt wird, und zum Ausgeben eines ersten und zweiten Probensignals, wenn dem Massenspektrometer ein erstes bzw. zweites der mindestens zwei Teilgase aus dem Gaschromatographen mittels der Gaszufuhrschnittstelle zugeführt wird. Eine Steuerungsvorrichtung ist dazu eingerichtet, die Kohlenstoff-, Stickstoff- und/oder Schwefelkonzentration anhand des ersten und zweiten Referenzsignals und des ersten und zweiten Probensignals zu ermitteln.

Eine Kleinstprobe im Sinne der Erfindung ist insbesondere eine Probe, die Kohlenstoff, Stickstoff und/oder Schwefel enthält und deren Probengewicht zwischen 12 und 120 µg liegt. Vorzugsweise enthält eine Kleinstprobe zwischen 2,5 und 14 µg Stickstoff. Vorzugsweise enthält eine Kleinstprobe zwischen 10 und 104,5 µg Kohlenstoff. Vorzugsweise enthält eine Kleinstprobe zwischen 0,2 und 1,5 µg Schwefel.

Ein Behandeln im Sinne der Erfindung ist insbesondere ein Umsetzen einer Probe, insbesondere in einen Probengasstrom, so dass beispielsweise einzelne, insbesondere flüchtige, Bestandteile der Probe in einem Gaschromatographen voneinander in Teilgase getrennt werden können. Ein Behandeln kann etwa ein Konfigurieren oder Vorbereiten der Probe auf eine Probengastrennung im Gaschromatographen sein.

Ein Aspekt basiert auf dem Ansatz, zumindest zwei flüchtige Bestandteile einer Probe, insbesondere Kleinstprobe, durch eine erste und eine zweite Behandlungseinrichtung zu leiten und durch dabei aufeinanderfolgend stattfindende chemische Reaktionen, insbesondere Oxidationen und Reduktionen, derart aufzubereiten, dass die Bestandteile, auch im Bereich von wenigen Mikrogramm, in einem Gaschromatographen getrennt werden können und mit den getrennten Bestandteilen, d.h. Teilgasen, eine Elementaranalyse durchgeführt werden kann. Die chemischen Reaktionen, insbesondere Oxidation und Reduktion, in der ersten Behandlungseinrichtung finden dabei in mehreren, vorzugsweise voneinander getrennten, Bereichen statt, insbesondere in einem ersten Oxidationsbereich, einem Reduktionsbereich und einem zweiten Oxidationsbereich. Die zumindest zwei Bestandteile der Probe können in der Weise im Wesentlichen vollständig, insbesondere mit in den Bereichen der ersten Behandlungseinrichtung und in der zweiten Behandlungseinrichtung angeordneten Reaktionsstoffen, reagieren. Dadurch ist die Trennung der zumindest zwei Bestandteile in einer Trennsäule des Gaschromatographen in mindestens zwei Teilgase möglich, wobei die Freigabe der Teilgase aus dem Gaschromatograph bzw. dessen einer Trennsäule zur Elementaranalyse in einem Massenspektrometer dann einfach über eine Temperaturregelung möglich ist.

Die erste und/oder zweite Behandlungseinrichtung ist bzw. sind dazu in bevorzugter Weise röhrenförmig ausgebildet und aus Quarzglas gefertigt. Die Probe kann hinter einer Öffnung der ersten Behandlungseinrichtung zum Einführen der Probe, insbesondere zwischen der Öffnung und dem ersten Oxidationsbereich, zumindest teilweise verdampft werden. Vorzugsweise weist die Vorrichtung zur Behandlung von Proben zu diesem Zweck eine Heizanordnung, beispielsweise einen um die röhrenförmige Behandlungseinrichtung gewundenen Heizdraht, insbesondere einen um die erste und zweite Behandlungseinrichtung angeordneten Hochtemperaturofen, auf.

Die erste Behandlungseinrichtung ist, beispielsweise über ein Glasröhrchen, mit der zweiten Behandlungseinrichtung verbunden, so dass die durch die zumindest teilweise Verdampfung der Probe freigesetzten Bestandteile der Probe nach deren Reaktion in den Oxidationsbereichen und dem Reduktionsbereich zuverlässig in die zweite Behandlungseinrichtung geleitet werden können.

Zum Einführen von insbesondere einer kleinen Menge der Probe, d.h. einer Kleinstprobe, in die erste Behandlungseinrichtung durch die Öffnung wird bevorzugt eine Zufuhreinrichtung verwendet, die beispielsweise mit einer Zufuhrvorrichtung, insbesondere einem Autosampler, koppelbar ist. Die Zufuhreinrichtung erlaubt ein präzises Leiten der durch die Zufuhrvorrichtung bereitgestellten Probemenge in die erste Behandlungseinrichtung, insbesondere durch Gravitation, wobei die Probe vor dem Austritt aus einer Austrittsöffnung der Zufuhreinrichtung in einem Heizbereich der Zufuhreinrichtung zumindest teilweise verdampft wird. Der Heizbereich kann beispielsweise durch eine Heizeinrichtung der Zufuhrvorrichtung definiert sein. Alternativ oder zusätzlich kann der Heizbereich auch durch eine Heizanordnung der Vorrichtung zur Behandlung von Proben definiert sein. Insbesondere kann die Austrittsöffnung der Zufuhreinrichtung bei der Verbindung mit der Vorrichtung zur Behandlung von Proben innerhalb der ersten Behandlungseinrichtung, etwa unterhalb der Öffnung der ersten Behandlungseinrichtung, angeordnet sein, um ein zuverlässiges Einführen von Proben in die Vorrichtung zur Behandlung von Proben zu ermöglichen. Nach der Einführung in die erste Behandlungseinrichtung, d.h. in der ersten Behandlungseinrichtung, aber noch während dem Durchfallen der Zufuhreinrichtung, kann die Probe in bevorzugter Weise durch die Heizanordnung in der Weise zumindest teilweise verdampft werden.

Insgesamt ermöglicht die Offenbarung eine besonders effiziente Elementaranalyse von Proben.

In einer bevorzugten Ausführungsform enthält der erste Oxidationsbereich Wolframoxid. Dadurch kann eine besonders zuverlässige erstmalige Oxidation der zumindest teilweise verdampften Probe, insbesondere von zumindest zwei flüchtigen Probenbestandteilen, erreicht werden.

In einer weiteren bevorzugten Ausführungsform enthält der Reduktionsbereich Kupferdraht. Vorzugsweise weist der hierbei verwendete Kupferdraht einen besonders hohen Reinheitsgrad, beispielsweise höher als 99 %, bevorzugt höher als 99,5%, insbesondere höher als 99,9 % auf. Dadurch lässt sich eine besondere zuverlässige erstmalige Reduktion der zumindest teilweise verdampften Probe, insbesondere von zumindest zwei flüchtigen Probenbestandteilen, erreichen.

In einer weiteren bevorzugten Ausführungsform enthält der zweite Oxidationsbereich Quarzsplitter oder versilbertes Kobaltoxid. Vorzugsweise werden Quarzsplitter eingesetzt, wenn unter Verwendung der zumindest teilweise verdampften Probe Isotopenverhältnisse der Probe von Kohlenstoff, Stickstoff und Schwefel in einem mit dem Gaschromatographen verbundenen Massenspektrometer ermittelt werden sollen. Alternativ kann versilbertes Kobaltoxid eingesetzt werden, wenn unter Verwendung der zumindest teilweise verdampften Probe Isotopenverhältnisse der Probe von Kohlenstoff und Stickstoff ermittelt werden sollen.

Die Quarzsplitter bzw. das versilberte Kobaltoxid ermöglichen eine vollständige Oxidation der zumindest teilweise verdampften Probe in der ersten Behandlungseinrichtung.

In einer weiteren bevorzugten Ausführungsform enthält die zweite Behandlungseinrichtung pulverförmigen Kupfer. Alternativ kann der Kupfer auch in Form von feinen Drähten vorliegen. Die Befüllung der zweiten Behandlungseinrichtung mit dem pulver- bzw. drahtförmigen Kupfer ermöglicht eine vollständige Reduktion der zumindest teilweise verdampften Probe in der zweiten Behandlungseinrichtung.

In einer weiteren bevorzugten Ausführungsform ist der erste Oxidationsbereich vom Reduktionsbereich und/oder der Reduktionsbereich vom zweiten Reduktionsbereich durch zumindest einen Trennbereich, in dem Quarzwolle angeordnet ist, getrennt. Alternativ oder zusätzlich können auch Quarzsplitter eingesetzt werden. Vorzugsweise ist auch der Reduktionsstoff zur zweimaligen Reduktion in der zweiten Behandlungseinrichtung, insbesondere das Kupferpulver bzw. die Kupferdrähte, zwischen Quarzwolle und/oder Quarzsplittern angeordnet. Der oder die Trennbereiche können insbesondere chemisch inerte und gasdurchlässige Stopfen bilden, welche die Oxidation- bzw. Reduktionsstoffe zur Reaktion mit der zumindest teilweise verdampften Probe in der ersten bzw. zweiten Behandlungseinrichtung zuverlässig positionieren.

In einer weiteren bevorzugten Ausführungsform weist die Vorrichtung, insbesondere die erste Behandlungseinrichtung, einen zwischen der Öffnung und dem ersten Oxidationsbereich angeordneten Probenfänger auf, der zur Aufnahme von feststofflichen Verdampfungsprodukten, beispielsweise Asche, der zumindest teilweise verdampften Probe eingerichtet ist. Vorzugsweise ist der Probenfänger zumindest teilweise aus Quarzglas gefertigt und becherförmig ausgebildet. Dabei kann der Probenfänger auch Quarzwolle aufweisen, die einen Boden des Probenfängers bildet. In diesem Fall schließt eine Außenseite des Probenfängers in bevorzugter Weise bündig mit einer Innenseite der ersten Behandlungseinrichtung ab. Die feststofflichen Verdampfungsprodukte können auf diese Weise zuverlässig aufgefangen werden, um eine Verunreinigung der Reaktionsstoffe in den Oxidationsbereichen und/oder den Reduktionsbereich zu verhindern. Gleichzeitig kann die zumindest teilweise verdampft Probe, d.h. die zumindest zwei flüchtigen Bestandteile der Probe, ungehindert durch die erste Behandlungseinrichtung geleitet werden.

In einer weiteren bevorzugten Ausführungsform weist die Vorrichtung eine Probenfängeraustauscheinrichtung auf, die dazu eingerichtet ist, den Probenfänger über die Öffnung in die erste Behandlungseinrichtung einzuführen oder aus der ersten Behandlungseinrichtung herauszuführen. Vorzugsweise weist der Probenfänger dabei einen Eingriff, insbesondere einen Durchgriff, auf, der zur Wechselwirkung mit der Probenfängeraustauscheinrichtung eingerichtet ist. Beispielsweise kann eine als Greifhaken ausgestaltete Probenfängeraustauscheinrichtung in den auf der Innenseite oder seitlich auf dem Probenfänger angeordneten Eingriff einhaken, um den Probenfänger mitsamt feststofflichen Verdampfungsprodukten aus der ersten Behandlungseinrichtung herauszuziehen, und den geleerten Probenfänger wieder in die erste Behandlungseinrichtung einzuführen. Dadurch kann die Reinigung der ersten Behandlungsrichtung verbessert und damit die zur zuverlässigen Elementaranalyse notwendige Präzision in der Probenbehandlung sichergestellt werden.

In einer weiteren bevorzugten Ausführungsform läuft ein Ende der Zufuhreinrichtung im Bereich der Austrittsöffnung konisch zu. Dabei kann eine Konusform des Endes in der Weise ausgestaltet sein, dass das Ende, insbesondere die Austrittsöffnung, der Zufuhreinrichtung zumindest teilweise durch die Öffnung der ersten Behandlungseinrichtung geführt und dadurch die Austrittsöffnung der Zufuhrvorrichtung mit der Vorrichtung zur Behandlung von Proben verbunden werden kann. Die Konusform des Endes der Zufuhreinrichtung ermöglicht es insbesondere, feststoffliche Verdampfungsprodukte der Probe zielgerichtet in den Probenfänger zu leiten. Dadurch kann die Vorrichtung zur Behandlung von Proben besonders sauber arbeiten.

In einer bevorzugten Ausführungsform weist die Zufuhreinrichtung entlang der Längsachse eine Länge zwischen 20 und 120 mm, vorzugsweise zwischen 30 mm und 110 mm, insbesondere zwischen 40 und 100 mm, und/oder im Bereich der Austrittsöffnung einen Durchmesser zwischen 1 und 12 mm, vorzugsweise zwischen 2 und 10 mm, insbesondere zwischen 3 und 8 mm, auf. Dadurch wird eine ausreichende Verdampfung der Probe beim Durchfallen der Zufuhreinrichtung bzw. ein besonders präzises Leiten der feststofflichen Verdampfungsprodukte in den Probenfänger der Vorrichtung zur Behandlung von Proben ermöglicht.

In einer weiteren bevorzugten Ausführungsform wird die Temperatur einer Trennsäule des Gaschromatographen zum Freigeben des zweiten der mindestens zwei Teilgase nach Messen des ersten Probensignals und Referenzsignals aus dem Gaschromatographen, insbesondere bis zu einer ersten vorgegebenen Zieltemperatur, erhöht. Vorzugsweise wird die Temperatur dabei gemäß einer ersten vorgegebenen Heizrate erhöht und/oder für eine erste vorgegebene Zeitdauer bei der ersten vorgegebenen Zieltemperatur gehalten, insbesondere bis das zweite der mindestens zwei Teilgase aus dem Gaschromatographen zur Analyse in das Massenspektrometer geleitet wurde. In bevorzugter Weise wird nach Messen des zweiten Probensignals und Referenzsignals die Temperatur weiter, insbesondere bis zu einer zweiten vorgegebenen Zieltemperatur und/oder gemäß einer zweiten vorgegebenen Heizrate erhöht und/oder für eine zweite vorgegebene Zeitdauer bei der zweiten vorgegebenen Zieltemperatur gehalten, insbesondere bis das dritte der mindestens zwei Teilgase aus dem Gaschromatographen in das Massenspektrometer geleitet wurde. Dadurch kann die Bestimmung von Isotopenverhältnissen der Probe, insbesondere Kleinstprobe, an einem Stück, d.h. mit nur einer zumindest teilweisen Verdampfung der Probe, durchgeführt werden.

Weitere Merkmale, Vorteile und Anwendungsmöglichkeiten der Erfindung ergeben sich aus der nachfolgenden Beschreibung im Zusammenhang mit den Figuren, in denen durchgängig dieselben Bezugszeichen für dieselben oder einander entsprechende Elemente der Erfindung verwendet werden. Es zeigen wenigstens teilweise schematisch:
- Fig. 1: ein Beispiel eines Systems zur quantitativen Elementaranalyse von Kohlenstoff, Stickstoff und/oder Schwefel;
- Fig. 2: ein Beispiel einer Vorrichtung zur Behandlung von Proben für eine quantitative Elementaranalyse;
- Fig. 3: ein Beispiel einer Zufuhrvorrichtung mit einer Zufuhreinrichtung;
- Fig. 4: ein Beispiel einer Zufuhreinrichtung;
- Fig. 5: ein Beispiel für eine Probenfängeraustauscheinrichtung; und
- Fig. 6: ein Beispiel eines Verfahrens zur quantitativen Elementaranalyse.

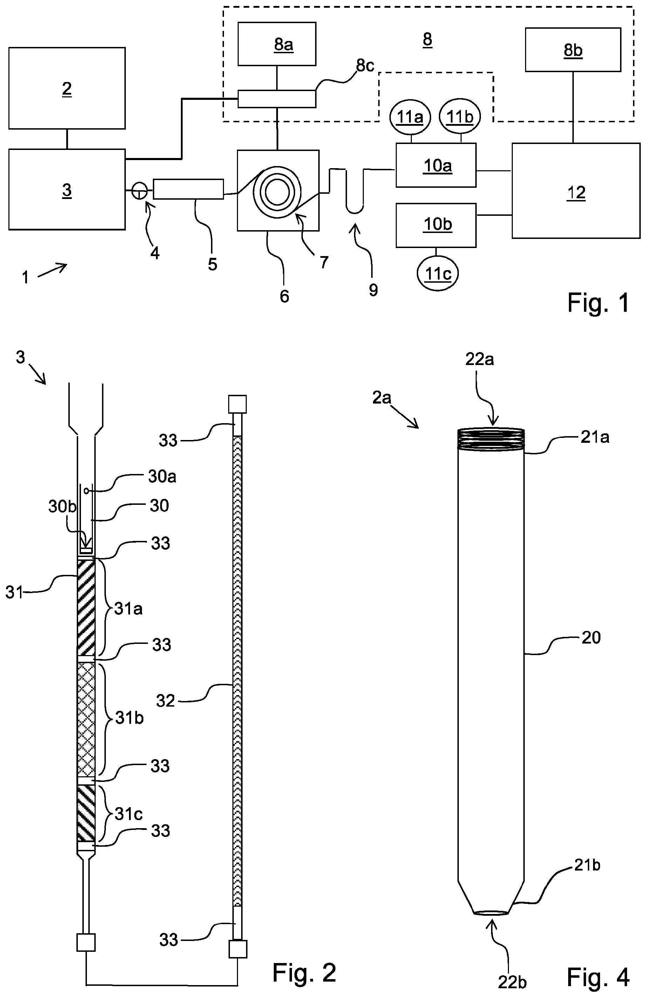

In Figur 1 ist ein Beispiel eines Systems zur quantitativen Elementaranalyse von Kohlenstoff, Stickstoff und/oder Schwefel mit einer Zufuhrvorrichtung 2, einer Vorrichtung zur Behandlung, insbesondere Umsetzung, von Proben 3, einer Teilervorrichtung 4, einer Wasserfalle 5, einem Gaschromatographen 6, einer Steuerungsvorrichtung 8, einer Gasfalle 9, Gaszufuhrschnittstellen 10a, 10b, Gasreservoirs 11a, 11b, 11c und einem Massenspektrometer 12, insbesondere einem Gasisotopenverhältnis-Massenspektrometer, gezeigt.

Die Probenzufuhr in die Vorrichtung 3 zur Behandlung von Proben erfolgt durch die Zufuhrvorrichtung 2, beispielsweise durch einen pneumatisch gesteuerten Autosampler, etwa vom Typ AS200 der Firma FISONS. Beim Zuführen wird die Probe erhitzt und dabei zumindest teilweise verdampft, so dass ein Probengasstrom, der insbesondere zumindest zwei flüchtige Bestandteile der Probe enthält, entsteht.

Die Behandlung der Probe, insbesondere der zumindest teilweise verdampften Probe, d.h. des Probengasstroms in der Umsetzungsvorrichtung 3 erfolgt in einer ersten und einer zweiten Reaktions- bzw. Behandlungseinrichtung (siehe Figur 2), insbesondere in zwei Quarzglasrohren verschiedener Durchmesser. Bei der Behandlung wird die zumindest teilweise verdampfte Probe zur gaschromatographischen Trennung im Gaschromatographen 6 aufbereitet.

Nach der Aufbereitung kann ein Teil der verdampften Probe in der Teilervorrichtung 4 abgezweigt werden. Die Teilervorrichtung 4 kann eine manuell zu bedienende oder automatisierte Einrichtung sein, um den Probengasstrom zu teilen. Ein Teil des Probengasstroms kann hierbei verworfen oder einer anderen Messeinrichtung (z.B. einem Sekundärdetektor) zugeführt werden.

Die Wasserfalle 5 weist vorzugsweise ein Kapillarrohr auf, das zum Anschluss an die Teilervorrichtung 4 und an den Gaschromatographen 6 geeignet ist. Die Wasserfalle 5 wird vorzugsweise mit einem Adsorbens, z.B. Phosphorpentoxid, gefüllt. Das Adsorbens kann insbesondere durch Quarzwolle in der Wasserfalle 5, insbesondere im Kapillarrohr, begrenzt und am Auslaufen gehindert werden.

Die Trennung der Probengase erfolgt über eine einzige gaschromatographische Trennsäule 7 im Gaschromatographen 6. Die Trennung der Gase wird vorzugsweise gemäß eines dynamisch geführten Temperaturprogramms durchgeführt, wobei die Temperatur in der Trennsäule 7, die Heizrate und/oder die Zeitdauern, mit der eine vorgegebene Temperatur gehalten wird, gemäß dem Temperaturprogramm gesteuert wird bzw. werden. Der Gaschromatograph 6 wird vorzugsweise mittels einer Steuerungsschnittstelle 8c einer Steuerungsvorrichtung 8 gesteuert. Das Temperaturprogramm kann beispielsweise durch ein Computerprogramm ausgeführt werden, welches von einer ersten Steuerungseinrichtung 8a ausgeführt wird. Die erste Steuerungseinrichtung 8a ist dabei vorzugsweise als Computer ausgebildet, der über die Steuerungsschnittstelle 8c die Probengastrennung im Gaschromatographen 6 aktiviert und steuert.

Die Trennsäule 7 ist vorzugsweise dazu eingerichtet, um die Gase N₂, CO₂ und SO₂ aus dem Probengasfluss zeitlich und quantitativ voneinander bei entsprechenden Zieltemperaturen zu trennen. Verschiedene Trennsäulenlängen, Innendurchmesser und stationäre Trennsäulenphasen sind hierbei möglich, um die im Wesentlichen vollständige Trennung der Gase sicherzustellen.

Über die Steuerungsschnittstelle 8c werden vorzugsweise die Signale zum Start der Vorrichtung 3 zum Umsetzen der Probe und zur Aktivierung der Gasfalle 9 weitergegeben. Insbesondere wird mit dem Signal zum Start der Vorrichtung 3 auch der Beginn der chromatographischen Probengastrennung im Gaschromatographen 6 ausgelöst.

In der, vorzugsweise automatisiert betriebenen, Gasfalle 9 werden die Probengase gesammelt. Die Gasfalle 9 kann mit Flüssigstickstoff zur Kühlung der Gase betrieben werden. Andere Arten der Kühlung sind aber ebenso möglich. Vorzugsweise weist die Gasfalle 9 eine Kühlfallenschleife auf, die beispielsweise durch eine Kapillare gebildet wird. Die Kühlfallenschleife weist vorzugsweise eine Länge von etwa 50 cm auf. Aber auch andere Längen der Kühlfallenschleife sind möglich.

Über die erste Gaszufuhrschnittstelle 10a können die Probengase in das Massenspektrometer 12 geleitet werden. Die erste Gaszufuhrschnittstelle 10a kann beispielsweise als ein pneumatisch gesteuertes "open split System", etwa vom Typ Conflo III der Firma Thermo Fisher Scientific, ausgeführt sein. Alternativ sind aber auch andere Gaszufuhrschnittstellen mit vergleichbarer Funktionalität denkbar.

Das Massenspektrometer 12 kann beispielsweise als Gasisotopenverhältnis-Massenspektrometer, etwa vom Typ Delta Plus Advantage der Firma Thermo Fisher Scientific, ausgebildet sein. Alternativ sind aber auch andere Gasisotopenverhältnis-Massenspektrometer denkbar.

Das Massenspektrometer 12 wird vorzugsweise von einer zweiten Steuerungseinrichtung 8b der Steuerungsvorrichtung 8 gesteuert. Die zweite Steuerungseinrichtung 8b ist beispielsweise als Computer ausgebildet und dazu eingerichtet, mittels Software das Massenspektrometer 12 zu aktivieren und zu steuern.

Für die Messungen im Massenspektrometer 12 können Referenzgase, insbesondere Stickstoff, Kohlenstoffdioxid und/oder Schwefeldioxid jeweils aus einem entsprechenden Stickstoffreservoir 11a, Kohlenstoffreservoir 11b bzw. Schwefelreservoir 11c über die erste Gaszufuhrschnittstelle 10a bzw. eine zweite Gaszufuhrschnittstelle 10b in das Massenspektrometer 12 geleitet werden. Vorzugsweise wird die Zuleitung der Referenzgase ebenfalls durch die Steuerungsvorrichtung 8, insbesondere die zweite Steuerungseinrichtung 8b, gesteuert.

Gemäß dem von der ersten Steuerungseinrichtung 8a ausgeführten Temperaturprogramm wird eine Starttemperatur im Gaschromatographen 6 so gewählt, dass das Kohlendioxid und das Schwefeldioxid aus dem Probengasfluss in der Trennsäule 7 verbleiben, bis ein erster Messvorgang zur Bestimmung von Stickstoff-Isotopenverhältnissen mittels des Massenspektrometers 12 abgeschlossen ist. Mit dem Einleiten eines zweiten Messvorgangs und einer vorgegebenen ersten Heizrate bis zum Erreichen einer ersten Zieltemperatur, die gegebenenfalls für eine vorgegebene erste Zeitdauer gehalten wird, wird dann das Kohlendioxid freigesetzt und Kohlenstoff-Isotopenverhältnisse gemessen. Bei weiterer Erhöhung der Temperatur durch eine zweite vorgegebenen Heizrate bis zu einer zweiten Zieltemperatur und gegebenenfalls dem Halten der zweiten Zieltemperatur für eine zweite vorgegebenen Zeitdauer wird dann das Schwefeldioxid von der Trennsäule freigesetzt und entsprechende Schwefel-Isotopenverhältnisse gemessen.

In Figur 2 ist ein Beispiel für eine Vorrichtung 3 zum Umsetzen von Proben gezeigt. Die Vorrichtung weist eine erste Reaktionseinrichtung 31 bzw. erste Behandlungseinrichtung 31 mit einem ersten Oxidationsbereich 31a, einem Reduktionsbereich 31b und einem zweiten Oxidationsbereich 31c sowie eine zweite Reaktionseinrichtung 32 bzw. zweite Behandlungseinrichtung 32 auf.

Die erste und zweite Reaktionseinrichtung 31, 32 sind vorzugsweise als Quarzglasrohre ausgebildet. Die erste Reaktionseinrichtung 31 ist vorzugsweise zum Anschluss an eine Zufuhrvorrichtung, insbesondere eine Zufuhreinrichtung (nicht gezeigt), eingerichtet. Die erste und zweite Reaktionseinrichtung 31, 32 sind dabei in bevorzugter Weise innerhalb einer Heizanordnung, beispielsweise einem Hochtemperaturofen, eingerichtet.

Die erste Behandlungseinrichtung 31 ist mit Reaktionsstoffen zur Probenoxidation im ersten Oxidationsbereich 31a und im zweiten Oxidationsbereich 31c bzw. zur primären Reduktion im Reduktionsbereich 31b befüllbar. Bevorzugtes Oxidationsmittel im ersten Oxidationsbereich 31a ist Wolframoxid. Als Reduktionsmittel werden vorzugsweise Kupferdrähte, insbesondere mit einem hohen Reinheitsgrad, verwendet. Im zweiten Oxidationsbereich 31b kann, insbesondere zum Bestimmung von Isotopenverhältnissen zwischen Kohlenstoff, Stickstoff und/oder Schwefel, beispielsweise Quarzsplittern angeordnet werden.

Zur Bestimmung von Isotopenverhältnissen zwischen Kohlenstoff und Stickstoff wird die untere Quarzsplitterfüllung des zweiten Oxidationsbereich 31c vorzugsweise gegen versilbertes Kobaltoxid ausgetauscht.

Die Chemikalien können für eine schnelle und vollständige Probenoxidation, d.h. des Probengasstroms bzw. der zumindest zwei flüchtigen Bestandteile der Probe, sorgen. Der erste Oxidationsbereich 31a, der Reduktionsbereich 31b und der zweite Oxidationsbereich 31c werden vorzugsweise durch Trennbereiche 33 voneinander getrennt. Die Trennbereiche 33 können mit Quarzwolle und/oder Quarzsplittern befüllt sein.

Die erste Reaktionsinrichtung 31 weist einen Probenfänger 30, insbesondere aus Quarzglas auf, der dazu eingerichtet ist, bei der Probenverdampfung übrig gebliebene feststoffliche Verdampfungsprodukte, insbesondere Asche, aufzufangen. Der Probenfänger 30 kann beispielsweise als Röhre ausgebildet sein und mit Quarzglaswolle verschlossen werden. Alternativ kann der Probenfänger 30 einen für die feststofflichen Verdampfungsprodukte undurchlässigen Probenfängerboden 30b aufweisen. Vorzugsweise wird die Länge des Probenfängers 30 und/oder die Wandstärke des Probenfängers 30 in Abhängigkeit der Probe, insbesondere der Probenmenge und/oder der Probeneigenschaften, ausgewählt.

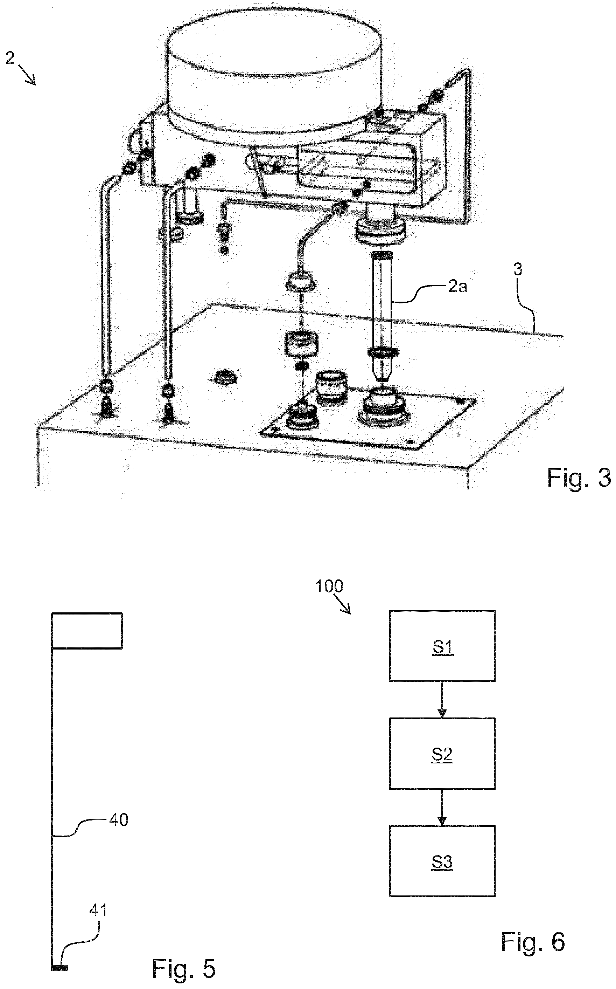

Vorzugsweise weist der Probenfänger 30 einen auf der Innenseite oder seitlich am Probenfänger 30 angeordneten Eingriff 30a auf, beispielsweise eine Öffnung, welcher den Tausch des Probenfängers 30 mittels einer Probenfängeraustauscheinrichtung (siehe Figur 5) ermöglicht.

Die zweite Reaktioneinrichtung 32 ist vorzugsweise zum Anschluss an die erste Reaktionseinrichtung 31 und an eine Teilervorrichtung (nicht gezeigt) eingerichtet. Die zweite Reaktionseinrichtung 32 ist zur Probenreduktion mit feinen Kupferdrähten befüllbar. Die Kupferdrahtfüllung kann durch Trennbereiche 33, in welchem beispielsweise Quarzglassplitter und/oder Quarzwolle angeordnet sind, begrenzt werden.

Vorzugsweise bestimmt das Volumen der zweiten Reaktionseinrichtung 32 die Reduktionseffizienz der Vorrichtung 3. Ein Durchmesser der zweiten Reaktionseinrichtung 32 kann beispielsweise etwa 4 mm betragen. Der Durchmesser kann ggf. aber auch geringer sein.

Figur 3 zeigt ein Beispiel einer Zufuhrvorrichtung 2, beispielsweise einen pneumatisch gesteuerten Autosampler, etwa vom Typ AS200 der Firma FISONS, mit einer Zufuhreinrichtung 2a. Die Zufuhrvorrichtung 2 ist außerhalb einer Vorrichtung 3 zur Behandlung von Proben, insbesondere außerhalb einer Heizanordnung der Vorrichtung 3, beispielsweise einem Hochtemperaturofen, angeordnet bzw. mit dieser verbindbar.

Die Zuführeinrichtung 2a ist dazu eingerichtet, die von der Zufuhrvorrichtung 2 bereitgestellte Probe, insbesondere durch zumindest einen Teil der Heizanordnung, etwa durch eine Gehäusewand der Heizanordnung, hindurch, in eine erste Behandlungseinrichtung (siehe Figur 2), insbesondere durch Ausnutzen der Schwerkraft, zu führen. Vorzugsweise fällt die Probe nach der Bereitstellung dabei mit Hilfe der Zuführeinrichtung 2a durch einen oder mehrere Heizbereiche, die in bevorzugter Weise von der Heizanordnung definiert werden, so dass die Probe erhitzt wird und verdampft. Dadurch entsteht ein Probengasstrom, der an einem der Zufuhrvorrichtung 2 gegenüberliegenden Ende der Zufuhreinrichtung 2a aus der Zuführeinrichtung 2a in die Vorrichtung zum Umsetzen von Proben eintritt.

Figur 4 zeigt ein Beispiel einer Zufuhreinrichtung 2a mit einem zylinderförmigen Körper 20. Die Zufuhreinrichtung 2a weist an einem ersten Ende 21a des Körpers 20, das bei Verwendung der Zufuhreinrichtung 2a einer Zufuhrvorrichtung (siehe Figur 3) zugewandt ist, eine Eintrittsöffnung 22a und an einem dem ersten Ende 21a gegenüberliegenden zweiten Ende 21b eine Austrittsöffnung 22b auf.

Durch die Eintrittsöffnung 22a kann die Probe von der Zufuhrvorrichtung in die Zuführeinrichtung 2a eingeführt werden, insbesondere fallen, so dass Probe beim Durchlaufen des Körpers 20 von einer Heizanordnung erhitzt und zumindest teilweise verdampft werden kann. Der dabei erzeugte Probengasstrom, d.h. die zumindest zwei flüchtigen Bestandteile der Probe, kann durch die Austrittsöffnung 22b aus- und in eine Vorrichtung zur Umsetzung von Proben (siehe Figur 2) eintreten.

Der Körper 20 ist vorzugsweise aus einem zwischen 40 und 120 mm langen Edelstahlrohr gefertigt und kann im Bereich des ersten Endes 21a ein Außengewinde zum Einschrauben in die Zufuhrvorrichtung aufweisen.

Im Bereich des zweiten Endes 21b verjüngt sich der Körper 20 konisch bis zur Austrittsöffnung 22b. Der Durchmesser der Austrittsöffnung 22b beträgt vorzugsweise zwischen 3 und 8 mm. Länge und Durchmesser des Körpers 20 ist der Probenumsetzungsgegebenheit anzupassen.

Figur 5 zeigt ein Beispiel für eine Probenfängeraustauscheinrichtung 40 zum Tausch eines Probenfängers einer Vorrichtung zum Umsetzen von Proben (siehe Figur 2). Die Probenfingeraustauscheinrichtung 40 weist einen Haken 41 auf, der in einen Eingrifft, insbesondere einen Durchgriff, des Probenfängers eingeführt werden kann, so dass der Probenfänger aus einer ersten Reaktionseinrichtung der Vorrichtung herausgeholt werden kann.

Figur 6 zeigt ein Beispiel eines Verfahrens 100 zur quantitativen Elementaranalyse von Kohlenstoff, Stickstoff und/oder Schwefel.

In einem ersten Verfahrensschritt S1 wird ein Stickstoff-Referenzgas in ein Massenspektrometer, insbesondere über eine erste Gaszufuhrschnittstelle, eingeleitet und Stickstoff-Referenzsignale, so genannte "Peak Center", gemessen. Dabei wird die maximale Signalstärke in Abhängigkeit von Konfigurationen des Massenspektrometers ermittelt. Mittels der Referenzsignale kann bei einer späteren Messung von Stickstoff-Isotopenverhältnissen die Signalstabilität und/oder Signalempfindlichkeit bei der entsprechenden Konfiguration des Massenspektrometers erhöht werden.

Im Rahmen des ersten Verfahrensschritts S1 kann ein Startsignal an eine Zufuhrvorrichtung ausgegeben werden, die daraufhin eine Probe bereitstellt und einer Vorrichtung zur Behandlung von Proben zugeführt. Dabei wird die Probe zumindest teilweise verdampft und ein Probengasstrom erzeugt. Das Startsignal kann ebenfalls ein Temperaturprogramm zur Probengastrennung in einem Gaschromatographen starten. Der umgesetzte Probengasstrom kann dadurch in einer Trennsäule des Gaschromatographen in einzelne Probengasbestandteile aufgespalten werden. Dabei werden insbesondere Kohlendioxid und Schwefeldioxid in der Trennsäule gehalten.

Vorzugsweise wird das Startsignal über eine Steuerungsschnittstelle an die Zufuhrvorrichtung und/oder die Vorrichtung zum Umsetzen von Proben ausgegeben.

Im Probengasstrom enthaltener Stickstoff wird über die erste Gaszufuhrschnittstelle dem Massenspektrometer zugeführt und ein entsprechendes Isotopenverhältnis, insbesondere unter Berücksichtigung der Stickstoff-Referenzsignale, gemessen.

In einem zweiten Verfahrensschritt S2 wird ein Kohlenstoff-Referenzgas in das Massenspektrometer, insbesondere über die erste Gaszufuhrschnittstelle, eingeleitet und Kohlenstoff-Referenzsignale gemessen. Anschließend wird, insbesondere gemäß des Temperaturprogramms, das in der Trennsäule gehaltene Kohlendioxid freigesetzt, dem Massenspektrometer zugeführt und ein entsprechendes Kohlenstoff-Isotopenverhältnis gemessen, insbesondere unter Berücksichtigung der Kohlenstoff-Referenzsignale. Dabei kann das freigesetzte Kohlendioxid mit einem Trägergas, insbesondere Helium, verdünnt werden.

In einem dritten Verfahrensschritt S3 wird ein Schwefel-Referenzgas wird in das Massenspektrometer, insbesondere über eine zweite Gaszufuhrschnittstelle, eingeleitet und Schwefel-Referenzsignale gemessen. Anschließend kann das in der Trennsäule gehaltene Schwefeldioxid freigesetzt und, insbesondere über die erste Gaszufuhrschnittstelle, ebenfalls in das Massenspektrometer geleitet werden. Dabei wird, ein Schwefel-Isotopenverhältnis, insbesondere unter Berücksichtigung der Schwefel-Referenzsignale, gemessen.

Gegebenenfalls kann während der drei Verfahrensschritte jeweils eine Gasfalle aktiviert werden, so dass Teile des Probengasstroms, insbesondere ungewollt aus der Trennsäule des Gaschromatographen austretender Kohlendioxid oder Schwefeldioxid, in der Gasfalle zurückgehalten werden.

### Bezugszeichenliste

- 1: System zur quantitativen Elementaranalyse von C, N und/oder S
- 2: Zufuhrvorrichtung
- 2a: Zufuhreinrichtung
- 3: Vorrichtung zur Behandlung von Proben
- 4: Teilervorrichtung
- 5: Wasserfalle
- 6: Gaschromatograph
- 7: Trennsäule
- 8: Steuerungsvorrichtung
- 8a: erste Steuerungseinrichtung
- 8b: zweite Steuerungseinrichtung
- 8c: Steuerungsschnittstelle
- 9: Gasfalle
- 10a: erste Gaszufuhrschnittstelle
- 10b: zweite Gaszufuhrschnittstelle
- 11a: Stickstoffreservoir
- 11b: Kohlenstoffreservoir
- 11c: Schwefelreservoir
- 12: Massenspektrometer

- 20: Körper
- 21a, 21b: erstes, zweites Ende
- 22a, 22b: Eintritts-, Austrittsöffnung

- 30: Probenfänger
- 30a: Eingriff
- 30b: Probenfängerboden
- 31: erste Reaktionseinrichtung
- 31a: erster Oxidationsbereich
- 31b: Reduktionsbereich
- 31c: zweiter Oxidationsbereich
- 32: zweite Reaktionseinrichtung
- 33: Trennbereiche

- 40: Probenfängeraustauscheinrichtung
- 41: Haken

- 100: Verfahren zur quantitativen Elementaranalyse von C, N und/oder S

- S1 - S3: Verfahrensschritte

## Patentansprüche

1. Verwendung einer Zufuhreinrichtung (2a) zum Einführen von Proben, insbesondere Kleinstproben, in eine Vorrichtung (1) zur Behandlung von Proben, insbesondere Kleinstproben, für eine Trennung in einem Gaschromatographen (6) zur Elementaranalyse bezüglich Kohlenstoff, Stickstoff und/oder Schwefel, mit -einer ersten Behandlungseinrichtung (31) mit einer Öffnung, in der eine der ersten Behandlungseinrichtung (31) über die Öffnung zugeführte Probe zumindest teilweise verdampft werden kann und die dazu eingerichtet ist, die zumindest teilweise verdampfte Probe in einem ersten Oxidationsbereich (31a) erstmalig zu oxidieren, in einem Reduktionsbereich (31b) erstmalig zu reduzieren und in einem zweiten Oxidationsbereich (31c) zweitmalig zu oxidieren, wobei der Reduktionsbereich (31b) zwischen dem ersten und zweiten Oxidationsbereich (31a, 31c) angeordnet ist, und -einer zweiten Behandlungseinrichtung (32), die mit der ersten Behandlungseinrichtung (31) verbunden und dazu eingerichtet ist, die in der ersten Behandlungseinrichtung (31) zumindest teilweise verdampfte Probe zweitmalig zu reduzieren, wobei die Zufuhreinrichtung (2a) eine Eintrittsöffnung (22a), über welche die Zufuhreinrichtung (2a) mit einer Zufuhrvorrichtung (2) zur Bereitstellung von Proben, insbesondere Kleinstproben, koppelbar ist, und eine Austrittsöffnung (22b), über welche die Zufuhreinrichtung (2a) mit der Vorrichtung (1) verbindbar ist, aufweist und dazu eingerichtet ist, eine mittels der Zufuhrvorrichtung (2) über die Eintrittsöffnung (22a) eingeführte Probe entlang einer Längsachse durch einen Heizbereich zu führen, so dass die Probe beim Durchfallen der Zufuhreinrichtung (2a) vor Austritt durch die Austrittsöffnung (22b) zumindest teilweise verdampft wird, und wobei die Zufuhreinrichtung (2a) in Abhängigkeit von der Probenmenge und/oder mindestens einer Probeneigenschaft aus mehreren Zufuhreinrichtungen mit verschiedenen Durchmessern und/oder verschiedenen Längen gewählt wird.

2. Verwendung einer Zufuhreinrichtung (2a) gemäß Anspruch 1, wobei ein zweites Ende (21b) der Zufuhreinrichtung (2a) im Bereich der Austrittsöffnung (22b) konisch zuläuft.

3. Verwendung einer Zufuhreinrichtung (2a) gemäß Anspruch 1 oder 2, wobei die Zufuhreinrichtung (2a) entlang der Längsachse eine Länge zwischen 20 und 120 mm, vorzugsweise zwischen 30 mm und 110 mm, insbesondere zwischen 40 und 100 mm, und/oder im Bereich der Austrittsöffnung (22b) einen Durchmesser zwischen 1 und 12 mm, vorzugsweise zwischen 2 und 10 mm, insbesondere zwischen 3 und 8 mm, aufweist.

## Claims

1. Use of a feed device (2a) for introducing samples, in particular very small samples, into an device (1) for treating samples, in particular very small samples, for separation in a gas chromatograph (6) for elemental analysis with respect to carbon, nitrogen and / or sulphur, wherein the device (1) for treating samples comprises
- a first treatment device (31) with an opening in which a sample which has been supplied to the first treatment device (31) via the opening can be vaporised, at least in part, and which is set up to oxidise the at least partially vaporised sample in a first oxidation region (31a) for the first time, to reduce it in a reduction region (31b) for the first time, and to oxidise it in a second oxidation region (31c) for a second time, wherein the reduction region (31b) is arranged between the first and second oxidation regions (31a, 31c), and
- a second treatment device (32) which is connected to the first treatment device (31) and which is set up to reduce, for a second time, the sample which has been vaporised, at least in part, in the first treatment device (31), wherein the feed device (2a) has an inlet opening (22a), via which the feed device (2a) can be coupled to a feed apparatus (2) for providing samples, in particular very small samples, and an outlet opening (22b) via which the feed device (2a) can be connected to the device (1), and is set up to guide, along a longitudinal axis through a heating region, a sample which has been introduced by means of the feed apparatus (2) via the inlet opening (22a), so that the sample is vaporised, at least in part, as it falls through the feed device (2a) and before exiting through the outlet opening (22b), and wherein the feed device (2a) is selected, as a function of the sample quantity and / or at least one sample property, from a plurality of feed devices with different diameters and / or different lengths.

2. Use of a feed device (2a) according to claim 1, wherein a second end (21b) of the feed device (2a) tapers in the region of the outlet opening (22b).

3. Use of a feed device (2a) according to claim 1 or 2, wherein the feed device (2a) has a length along the longitudinal axis of between 20 mm and 120 mm, preferably between 30 mm and 110 mm, in particular between 40 mm and 100 mm, and / or a diameter in the region of the outlet opening (22b) of between 1 mm and 12 mm, preferably between 2 mm and 10 mm, in particular between 3 mm and 8 mm.

## Revendications

1. Utilisation d'un équipement d'alimentation (2a) pour l'introduction d'échantillons, en particulier d'échantillons miniaturisés, dans un dispositif (1) de traitement d'échantillons, en particulier d'échantillons miniaturisés, pour une séparation dans un chromatographe en phase gazeuse (6) pour l'analyse élémentaire relative au carbone, à l'azote et/ou au soufre, comprenant
- un premier équipement de traitement (31) avec une ouverture dans laquelle un échantillon fourni au premier équipement de traitement (31) via l'ouverture peut être au moins partiellement vaporisé et qui est conçu pour oxyder une première fois l'échantillon au moins partiellement vaporisé dans une première zone d'oxydation (31a), pour le réduire une première fois dans une zone de réduction (31b) et pour l'oxyder une seconde fois dans une seconde zone d'oxydation (31c), dans laquelle la zone de réduction (31b) est agencée entre la première et la seconde zone d'oxydation (31a, 31c), et
- un second équipement de traitement (32) qui est relié au premier équipement de traitement (31) et qui est conçu pour réduire une seconde fois l'échantillon au moins partiellement vaporisé dans le premier équipement de traitement (31), dans laquelle l'équipement d'alimentation (2a) présente une ouverture d'entrée (22a) via laquelle l'équipement d'alimentation (2a) peut être couplé à un dispositif d'alimentation (2) pour la fourniture d'échantillons, en particulier d'échantillons miniaturisés, et une ouverture de sortie (22b) via laquelle l'équipement d'alimentation (2a) peut être relié au dispositif (1) et est conçu pour guider un échantillon introduit au moyen du dispositif d'alimentation (2) via l'ouverture d'entrée (22a) le long d'un axe longitudinal à travers une zone de chauffage, de sorte que l'échantillon soit au moins partiellement vaporisé en cas d'échec de l'équipement d'alimentation (2a) avant la sortie par l'ouverture de sortie (22b), et dans laquelle l'équipement d'alimentation (2a) est sélectionné en fonction de la quantité d'échantillons et/ou d'au moins une propriété d'échantillon parmi plusieurs équipements d'alimentation avec des diamètres différents et/ou des longueurs différentes.

2. Utilisation d'un équipement d'alimentation (2a) selon la revendication 1, dans laquelle une seconde extrémité (21b) de l'équipement d'alimentation (2a) se termine en forme de cône dans la zone de l'ouverture de sortie (22b).

3. Utilisation d'un équipement d'alimentation (2a) selon la revendication 1 ou 2, dans laquelle l'équipement d'alimentation (2a) présente le long de l'axe longitudinal une longueur entre 20 et 120 mm, de préférence entre 30 mm et 110 mm, en particulier entre 40 et 100 mm, et/ou dans la zone de l'ouverture de sortie (22b) un diamètre entre 1 et 12 mm, de préférence entre 2 et 10 mm, en particulier entre 3 et 8 mm.
